# EUROPEAN PATENT APPLICATION

(11) **EP 2 444 805 A2**
(43) Date of publication of application: **25.04.2012**
(21) Application number: 11183171.5
(22) Date of filing: 21.01.2005
(51) Int. Cl.: G01N 33/53

(54) **Detection of mesothelin-/megakaryocyte potentiating factor-related peptides in peritoneal fluid for assessment of the peritoneum and the peritoneal cavity**

(30) Priority: 21.01.2004 US 538072 P
(62) Divisional of application: 05712014.9
(71) Applicant: Fujirebio America, Inc., Wilmington, DE 19808 (US)
(72) Inventor: O'Shannessy, Daniel J., Limerick PA Pennsylvania 19468 (US); Sardesai, Niranjan Y., North Wales PA Pennsylvania 19454 (US); Sommers, Elizabeth B., Coatesville PA Pennsylvania 19320 (US)
(74) Representative: Olgemöller, Luitgard Maria

(57) **Abstract**

The invention relates to methods and kits for assessing occurrence in patient mesothelial fluid of peptides having amino acid sequences related to those of mesothelin, megakaryocyte potentiating factor, and other peptides that have been associated with occurrence in the serum of mesothelioma patients. The methods and kits can be used to monitor the biochemical or pathological status of a component of the corresponding mesothelial cavity in a patient, to predict development of such a pathological status in an otherwise asymptomatic patient, or to assess the efficacy of a therapeutic method.

## Description

### BACKGROUND OF THE INVENTION

The invention relates generally to monitoring biochemical status of the peritoneal cavity, fluids therein, and the integrity of the peritoneal membrane.

Mesothelial membranes line various body cavities, such as the peritoneal cavity, the pleural cavity, and the pericardial cavity. Under certain circumstances, cells of mesothelial origin produce mesothelin, and mesothelin protein is sometimes secreted or shed from cells of mesothelial membranes.

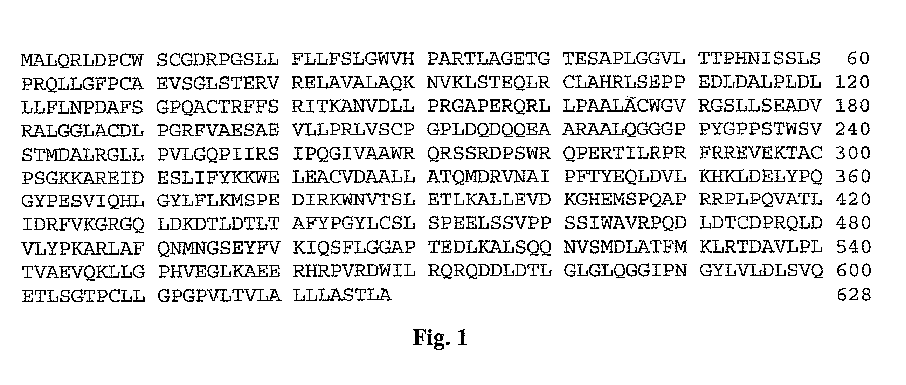

Mesothelin is a protein which is expressed on the surface of at least mesothelial cells, mesotheliomas, some squamous cell carcinomas, and ovarian cancers (Chang et al., ., 1996, Proc. Natl. Acad. Sci. USA 93:136-140). Mesothelin has been described in the literature (e.g., U.S. Patent No. 6,083,502). Antibodies that bind with mesothelin have also been described. For example, a monoclonal antibody designated K1 is described in U.S. Patent No. 5,320,956, and is secreted by the hybridoma that was deposited by others with the American Type Culture Collection (ATCC; 10801 University Boulevard; Manassas, VA 20110-2209; USA) as accession number HB 10570. Antibody K1 was found to bind with an approximately 40 kilodalton protein found on the surface of ovarian cancer and other cells (Chang et al., 1996, Proc. Natl. Acad. Sci. USA 93(1):136-140). That protein, designated mesothelin, is derived from a larger (approximately 69 kilodalton) precursor protein for which the sequence is shown in Fig. 1. Scholler et al. (1999, Proc. Natl. Acad. Sci. USA 96(20):11531-11536) recognized that an antigen having an amino acid sequence highly similar to the amino terminal sequences of mesothelin and of megakaryocyte potentiating factor (MPF) occurs in the serum of many patients afflicted with ovarian carcinoma. Scholler proposed that this antigen could be a useful serum marker for diagnosing ovarian carcinoma.

A useful diagnostic test should not only be able to detect occurrence of mesothelin and related proteins in a patient, it is also preferably inexpensive, non-invasive, and easy to use. Diagnostic tests that use blood or serum as a testing substrate require that blood be drawn from a patient to be screened, and sometimes require further separation, purification, or other treatment of the withdrawn blood sample. Drawing blood must be performed by a trained health care worker, is painful and invasive for the patient, requires specialized equipment and reagents for storage and transportation of blood samples, and carries the risk of transmission of blood-borne pathogens. By contrast, fluid samples are obtained from many patients who exhibit fluid buildup (effusion) within a body cavity lined by a mesothelial membrane such as the peritoneum, the pleural membranes, and the pericardium. For these reasons, diagnostic testing using mesothelial cavity samples as a testing substrate can be preferable to or synergistic with testing done with blood samples.

### BRIEF SUMMARY OF THE INVENTION

The present invention provides a diagnostic test that can be performed using a fluid sample obtained from a mesothelial cavity in order to monitor the biochemical status of the cavity, organs or tissues within the cavity, or the mesothelium lining the cavity (collectively, to monitor "mesothelial cavity status"). By way of example, the test can ,be performed using peritoneal fluid to assess the status of peritoneal organs, peritoneal fluid, and the peritoneum.

The invention relates to a method of monitoring mesothelial cavity status in a human patient. The method comprises assessing occurrence of a mesothelin/megakaryocyte potentiating factor family (MMPFF) peptide in a mesothelial fluid obtained from the patient. Occurrence of the MMPFF peptide in the mesothelial fluid is an indication that the patient is afflicted with mesothelioma.

In one embodiment, the MMPFF peptide is assessed in the patient's mesothelial fluid by contacting the mesothelial fluid (or centrifuged mesothelial fluid) with a first antibody that binds specifically with the MMPFF peptide. By assessing whether the MMPFF peptide has bound with the first antibody, occurrence of the MMPFF peptide in the patient's mesothelial fluid can be assessed. For example, the first antibody can be bound to a substrate, such as a plastic multi-well plate of the type adapted for automated analysis in a robotic apparatus. Binding of the MMPFF peptide and the first antibody can, for example, be assessed by contacting the first antibody with a second antibody that binds specifically with the MMPFF peptide and assessing co-localization of the first and second antibodies. The second antibody can be detectably labeled, such as with a compound selected from the group consisting of an enzyme, a radionuclide, a fluorophore, and a chromophore.

By way of example, the method can comprise contacting a plate having an anti-MMPFF peptide 'capture' antibody bound thereto with mesothelial fluid obtained from a patient. The mesothelial fluid can, optionally, be incubated with the plate for a period such as 10 minutes or an hour. The plate can then be contacted with a biotinylated second antibody that binds with an epitope of the MMPFF peptide than does the capture antibody. A streptavidin-linked enzyme can be bound to the biotinylated antibody to enable its detection in the presence of a chromogenic substrate (e.g., 3,3',5,5'-tetramethylbenzidine, which is a chromogenic substrate of horseradish peroxidase). Of course, the second antibody could instead be fluorescently labeled, radiolabeled, or otherwise detectably labeled.

In another embodiment of the method of monitoring mesothelial cavity status, the first antibody can be contacted with a labeled substrate thereof contacting the first antibody with the mesothelial fluid. By comparing the amount of labeled ligand that binds with the first antibody that has been contacted with the mesothelial fluid with the amount of labeled ligand that binds with an equivalent amount of the first antibody that has not been contacted with the mesothelial fluid, one can assess how much of MMPFF from the mesothelial fluid has bound with the first antibody.

The kits and methods described herein can be used to detect a variety of MMPFF peptides, including naturally occurring MMPFF peptides (i.e., both full length proteins such as mesothelin and fragments of such proteins that are naturally produced in the bodies of patients) and synthetically produced MMPFF peptides. The amino acid sequence of the MMPFF peptide should comprise at least 10 (20, 50, or 200) consecutive residues of a sequence selected from the group consisting of SEQ ID NOs: 1-5. Alternatively, the MMPFF peptide can comprise a portion of at least 20 consecutive amino acid residues, wherein the amino acid sequence of the portion is at least 90% (or 95%) identical to 20 consecutive residues of a sequence selected from the group consisting of SEQ ID NOs: 1-5.

The kits and methods disclosed herein can be used in conjunction with known or hereafter developed kits and methods for assessing occurrence (e.g., in mesothelial fluid or serum) other markers of pathological status of tissues or organs within the corresponding mesothelial cavity. Assessment of occurrence of MMPFF peptide in a patient's mesothelial fluid can likewise be performed in conjunction with assessment of the MMPFF peptide in the patient's scrum.

Substantially the same kits and methods can be used to assess the likelihood that a patient will develop a pathology of an organ or tissue of within a mesothelial cavity or of the mesothelial membrane lining the cavity. That is, occurrence of an MMPFF peptide in mesothelial fluid obtained from a patient is an indication that the patient is more likely to develop such pathology than an otherwise identical patient in whose mesothelial fluid the MMPFF does not occur.

Substantially the same kits and methods can also be used to assess the efficacy of a therapy for a pathological condition of a tissue or organ within a mesothelial cavity or for a pathological condition of a mesothelial membrane in a patient. The amount of an MMPFF peptide in mesothelial fluid obtained from the patient following the therapy can be compared with the amount of the MMPFF in her mesothelial fluid before the therapy. A lower amount of the MMPFF peptide in the mesothelial fluid following the therapy is an indication that the therapy is efficacious. Similarly, a greater amount of the MMPFF peptide in the mesothelial fluid following the therapy is an indication that the therapy is not efficacious. Such kits and methods can be used to compare the efficacy of various therapeutic agents or regimens.

In another aspect, the invention relates to a kit for assessing occurrence of an MMPFF peptide in mesothelial fluid obtained from a patient. The kit comprises a first agent (e.g., an antibody) for binding the MMPFF peptide and an instructional material that describes contacting mesothelial fluid with the first agent. The format of the kit is not critical - many standard formats can be used, such as ELISA, latex bead aggregation, and surface plasmon resonance formats. The kit can include an MMPFF peptide for use as a positive control. Of course, the kit can also include reagents for assessing other known markers of pathological conditions of mesothelial membranes or tissues or organs within mesothelial cavities.

### BRIEF SUMMARY OF THE SEVERAL VIEWS OF THE DRAWINGS

In all figures comprising amino acid sequences, standard single-letter amino acid codes are used to represent amino acid residues.

Figure 1 is the amino acid sequence (SEQ ID NO: 1) of mesothelin precursor protein. This sequence is the same as that listed in GENBANK® accession number 2207386A and reported in Chang et al., 1996, Proc. Natl. Acad. Sci. USA 93(1):136-140.

Figure 2 is the amino acid sequence (SEQ ID NO: 2) of megakaryocyte potentiating factor (MPF) precursor protein. This sequence is the same as that listed in GENBANK® accession number NP_005814 and reported in Yamaguchi et al., 1994, J. Biol. Chem. 269(2):805-808.

Figure 3 is a partial amino acid sequence (SEQ lD NO: 3) of a soluble variant form of MPF precursor protein listed in GENBANK® accession number AF180951 and reported in Scholler et al., 1999, Proc. Natl. Acad. Sci. USA 96(20):11531-11536.

Figure 4, comprising Figures 4A and 4B, is a comparison of residues 294-628 of SEQ ID NO: 1 and a nearly identical portion of SEQ ID NO: 2. Residue numbers are listed in the right margin. "MST" means mesothelin. "MPF" means megakaryocyte potentiating factor. In this alignment, identical amino acid residues are indicated by a vertical line, a conservative amino acid substitution is indicated by a cross (+), and dashes (-) indicate a gap inserted into SEQ ID NO: 2 for the purpose of aligning the sequences.

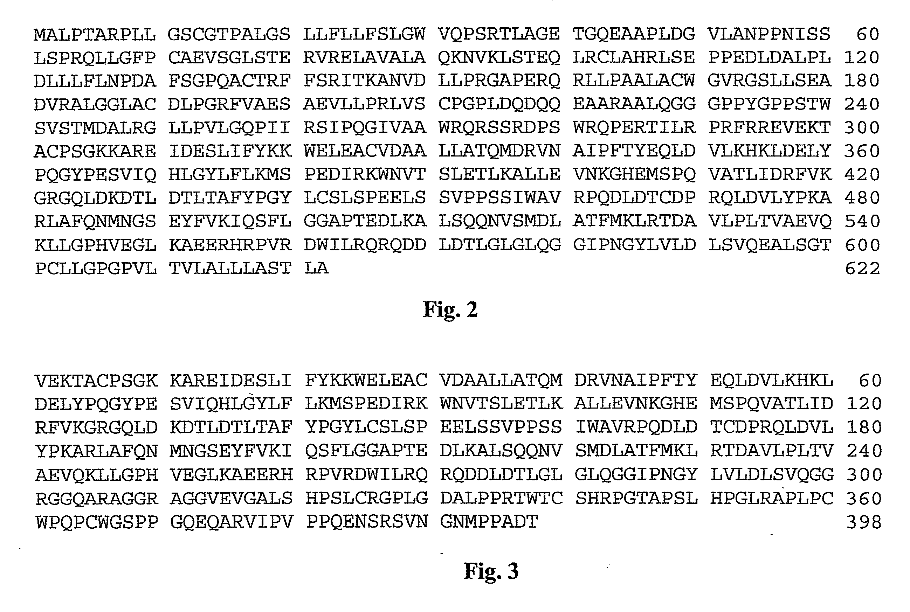

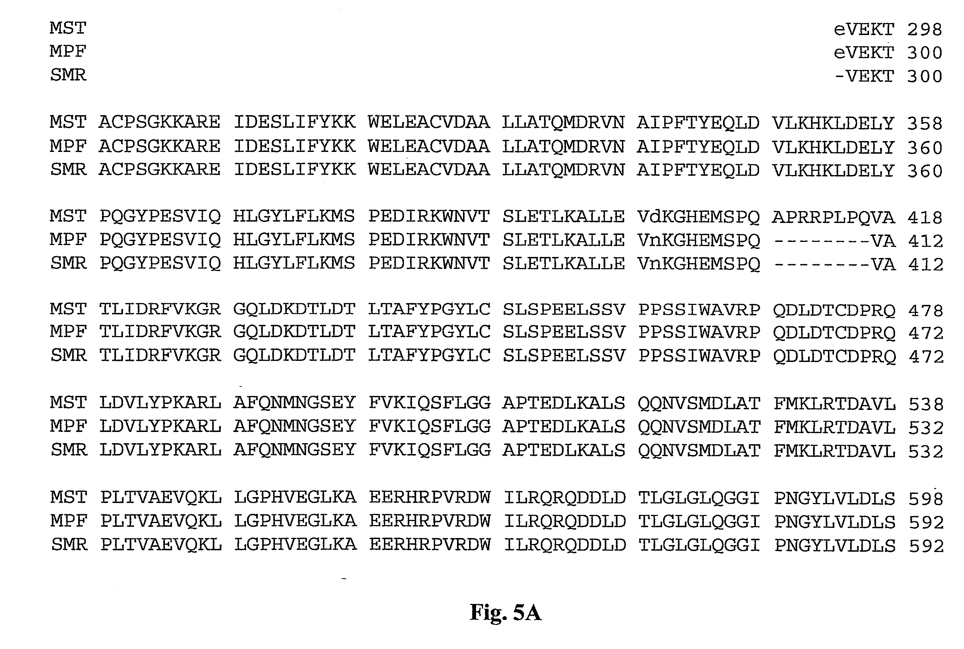

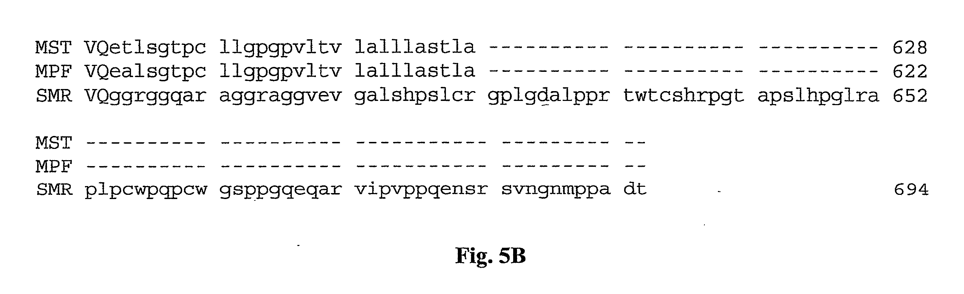

Figure 5, comprising Figures 5A and 5B, is a comparison of similar portions of SEQ ID NOs: 1-3. Residue numbers are listed in the right margin. "MST" means mesothelin. "MPF" means megakaryocyte potentiating factor. "SMR" means the soluble variant form of MPF for which the sequence is listed in Fig. 3. In this alignment, residues that are identical in all three sequences are represented by upper case single-letter codes, residues that are not identical in all three sequences are represented by lower case single-letter codes, and gaps inserted for the purpose of aligning the sequences are represented by dashes (-).

Figure 6 comprises Figures 6A and 6B. Figure 6A is an amino acid sequence (SEQ ID NO: 4) that includes preferred epitopes for generation of antibodies described herein. Figure 6B is another amino acid sequence (SEQ ID NO: 5) that includes preferred epitopes for generation of antibodies described herein. In each of SEQ ID NOs: 4 and 5, the underlined, bold X represents any amino acid residue, preferably being either aspartate or asparagine. Other underlined, bold residues in Figure 6A represent residues that are not present in the sequence shown in Figure 6B.

Figure 7 is a graph that indicates peritoneal fluid MMPFF peptide levels in peritoneal dialysis samples described in Example 2.

### DETAILED DESCRIPTION OF THE INVENTION

The invention relates to the discovery that one or more polypeptides having significant amino acid sequence similarity with a family of proteins that includes mesothelin, megakaryocyte potentiating factor (MPF), and a soluble variant of MPF occurs in the mesothelial fluid of patients afflicted with pathological mesothelial membranes or with pathological conditions of tissues, organs, or fluids within a mesothelial (e.g., peritoneal, pleural, or pericardial) cavity. These mesothelial fluid polypeptides bind specifically with antibodies that are raised against proteins of that family. Occurrence of these polypeptides in a patient's mesothelial fluid is diagnostic that the patient is afflicted with such a pathological condition. The concentration or occurrence of the polypeptides can also be used to monitor the biochemical status of a mesothelial cavity, such as a fluid, tissue, or organ within the cavity or the mesothelial membrane lining the cavity. The amount of the polypeptides decreases with effective treatment of such pathological conditions. Thus, the mesothelial fluid polypeptides can also be used to assess the efficacy of various therapeutic methods.

Definitions

As used herein, each of the following terms has the meaning associated with it in this section.

A mesothelin/megakaryocyte potentiating factor family peptide ("MMPFF peptide") is a polypeptide that has an amino acid sequence that either (i) comprises at least 10 consecutive residues of a sequence selected from the group consisting of SEQ ID NOs: 1-5 or (ii) comprises a portion of at least 20 consecutive amino acid residues wherein the amino acid sequence of the portion is at least 90% (preferably at least 95% or 100%) identical to 20 consecutive residues of a sequence selected from the group consisting of SEQ ID NOs: 1-5.

An "antibody" refers collectively and individually to immunoglobulin molecules and immunologically active portions of immunoglobulin molecules, i.e., molecules that contain an antigen binding site which specifically binds an MMPFF peptide. A molecule which specifically binds with an MMPFF peptide is a molecule which binds the peptide, but does not substantially bind other molecules in a sample (e.g., a mesothelial fluid sample) which naturally contains the polypeptide. Examples of immunologically active portions of immunoglobulin molecules include F(ab) and F(ab')₂ fragments which can be generated by treating the antibody with an enzyme such as papain or pepsin, respectively. Either or both of polyclonal and monoclonal antibodies can be used in the methods and kits described herein.

The term "monoclonal antibody" refers to a population of antibody molecules that contain only one species of an antigen binding site capable of immunoreacting with a , I particular epitope.

The term "labeled," with regard to an antibody or a peptide, includes direct labeling of the peptide or antibody by coupling (i.e., physically linking) a detectable substance to the peptide or antibody, as well as indirect labeling of the peptide or antibody by coupling it with another reagent that is directly labeled. Examples of indirect labeling include detection of a primary antibody using a fluorescently labeled secondary antibody and end-labeling of a peptide with biotin such that it can be detected with fluorescently labeled streptavidin.

An "instructional material" is a publication, a recording, a diagram, or any other medium of expression which can be used to communicate how to use a kit described herein, information for interpreting occurrence of an MMPFF in mesothelial fluid as it relates to the biochemical status, pathological state, or both, of a mesothelial cavity component (e.g., a fluid, tissue, or organ contained within the cavity) or of a mesothelial cavity membrane (e.g., the peritoneum, a pleural membrane, or the pericardium). The instructional material of the kit of the invention can, for example, be affixed to a container which contains a kit of the invention or be shipped together with a container which contains the kit. Alternatively, the instructional material can be shipped separately from the container with the intention that the instructional material and the kit be used cooperatively by the recipient.

A "mesothelial" cavity is a body cavity lined with a membrane of mesothelial origin, such as the peritoneal cavity, the pleural cavities, and the pericardial cavity.

. A "component of a mesothelial cavity" means the membrane lining the cavity, a fluid contained within the cavity, or a tissue or organ within the cavity.

A "mesothelial fluid" means a fluid which either naturally occurs in the cavity (e.g., peritoneal fluid) or is artificially introduced into the cavity (e.g., a peritoneal washing or dialysis fluid).

Detailed Description

Soluble mesothelin-related peptides (SMRP) were first described as a serum marker for ovarian cancer and more generally as a serum marker for mesothelial cell related cancers. Indeed, recent reports indicate elevated levels of SMRP in serum samples taken from mesothelioma patients. The present invention demonstrates that SMRP can be detected in mesothelial cavity fluids of human patients and further, the presence of elevated levels of SMRP in these bodily fluids is indicative of the pathological and biochemical components of the corresponding cavity (i.e., mesothelial membrane and the fluid, tissue, and organs contained within it).

It has been discovered that one or more polypeptides having significant amino acid sequence similarity with a family of proteins (the mesothelin/megakaryocyte potentiating factor family; "MMPFF") occurs in the mesothelial fluid of patients. These mesothelial fluid peptides can be detected, for example with an antibody raised against a protein of the MMPFF. Occurrence of such peptides in a patient's mesothelial fluid indicate the biochemical and pathological status of the components of the corresponding mesothelial cavity of the patient. By way of example, the cancerous state of the mesothelial membrane lining the cavity or of one or more tissues or organs within the cavity can be assessed. Likewise, the health and viability of the mesothelial cell lining can be assessed using the methods described herein.

Diagnostic Methods

The invention includes a method of diagnosing the biochemical and pathological status of the components of a mesothelial cavity in a patient. The method comprises assessing occurrence of a mesothelin/megakaryocyte potentiating factor family (MMPFF) peptide in mesothelial fluid obtained from the patient. Occurrence of the MMPFF peptide in the mesothelial fluid is an indication of the biochemical and pathological status of the components of the corresponding mesothelial cavity.

The method used to assess occurrence of the MMPFF peptide in the patient's mesothelial fluid is not critical. Any method that can be used to assess whether the peptide is present or absent is suitable. Methods capable of assessing the quantity (e.g., concentration) of the MMPFF peptide in the mesothelial fluid are preferable in some embodiments, particularly when assessments made over a period of time are to be compared (e.g., as when assessing the efficacy of a regimen of anti-cancer therapy).

In one embodiment, occurrence of the MMPFF peptide in the patient's mesothelial fluid is assessed by contacting the mesothelial fluid with an antibody that binds specifically with the MMPFF peptide and assessing whether the MMPFF peptide has bound with the first antibody.

Numerous MMPFF-binding antibodies are known in the art. For example, the antibody described in U.S. Patent No. 5,320,956 and secreted by the hybridoma deposited with the ATCC as accession no. HB 10570 can be used. That antibody binds specifically with an approximately 40 kilodalton portion of mesothelin protein, as described by Chang et al. (1996, Proc. Natl. Acad. Sci. USA 93(1):136-140). Other suitable antibodies have been described by Scholler et al. (1999, Proc. Natl., Acad. Sci. USA 96(20):11531-11536) and in PCT publication number WO 00/50900, wherein such antibodies are designated OV569, 4H3, 3G3, and 1A6.

In diagnostic kits and methods that use two anti-MMPFF peptide antibodies, the two antibodies preferably do not compete to bind the same epitope. By way of example, the antibody designated OV569 by Scholler et al. binds at an epitope which is independent of the epitopes bound by the antibodies designated 4H3, 3G3, and 1A6. Thus, for example, OV569 can be bound to a substrate and used as a 'capture' antibody to bind MMPFF peptide present in a patient's mesothelial fluid under appropriate antibody-binding conditions, and one of antibodies 4H3, 3G3, and 1A6 can be detectably labeled and contacted with the 'capture' antibody after contacting the 'capture' antibody with the patient's mesothelial fluid. Binding of the detectably labeled antibody with the 'capture' antibody indicates that the MMPFF peptide was present in the patient's mesothelial fluid. Analysis of the amount of detectable label co-localized with the 'capture' antibody can indicate the quantity of the peptide that was present.

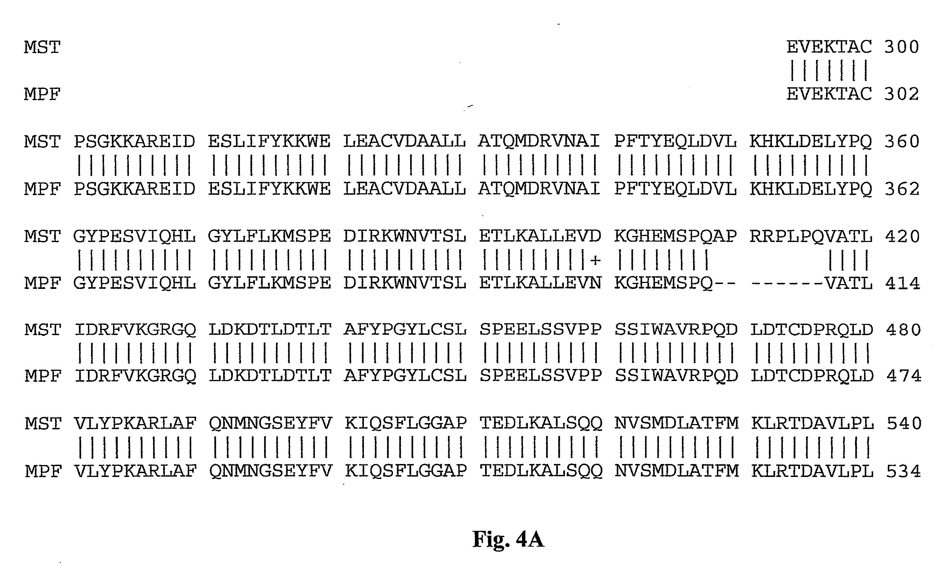

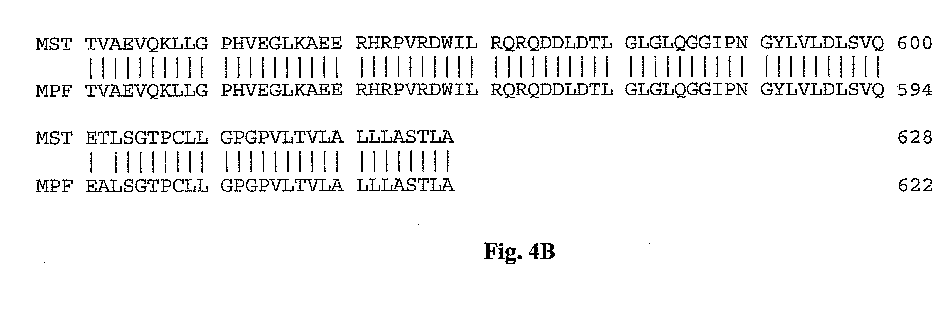

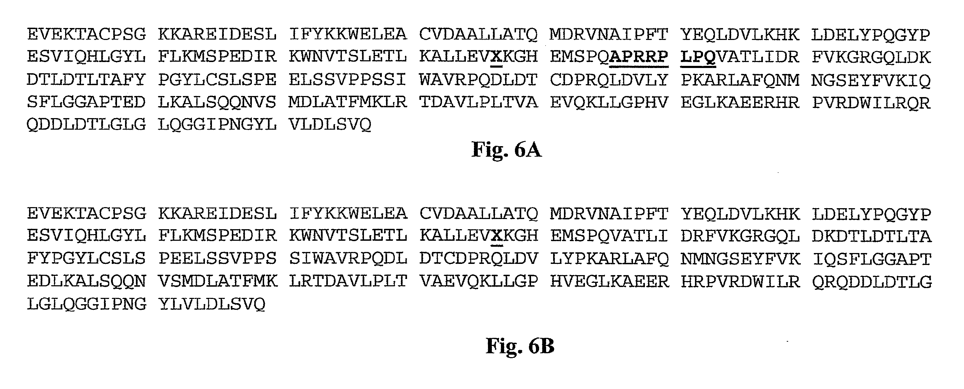

As described herein, portions of MMPFF proteins share significant amino acid sequence homology. Figs. 1-3 show at least portions of the amino acid sequences of three MMPFF proteins. Figure 4 and 5 show alignments of those sequences, indicating the regions of greatest sequence homology among these proteins. From these figures, it is apparent that the sequences shown in Figs. 6A and 6B (SEQ ID NOs: 4 and 5, respectively) represent useful portions of MMPFF peptides. Antibodies raised against all or a portion (e.g., 10, 20, 50, or 200 consecutive residues) of either of these sequences can be expected to bind specifically with a broad range of MMPFF peptides, including those that occur in the mesothelial fluids of patients.

An MMPFF (e.g., one described herein or in the prior art), or a fragment thereof, can be used as an immunogen to generate antibodies using standard techniques for polyclonal and monoclonal antibody preparation. A fragment of a full-length MMPFF, when used as an immunogen, should comprise at least 10 (preferably 12, 15, 20, 50,100, or 20 or more) amino acid residues of the amino acid sequence of any of SEQ ID NOs: 1-5 or the amino acid sequence of another MMPFF member.

Appropriate MMPFF peptides for generation of antibodies useful in the kits and methods described herein comprise a portion of at least 20 consecutive amino acid residues that is at least 90% (preferably at least 95% or 100%) identical to 20 consecutive residues of a sequence selected from the group consisting of SEQ ID NOs: 1-5 (preferably either SEQ ID NO: 4 or SEQ ID NO: 5).

Methods of generating antibodies are well known and only briefly summarized here. An immunogen typically is used to prepare antibodies by immunizing a suitable (i.e., immunocompetent) subject such as a rabbit, goat, mouse, or other mammal or vertebrate. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Polyclonal antibodies can be prepared as described above by immunizing a suitable subject with a polypeptide of the invention as an immunogen. The antibody titer in the immunized subject can be monitored over time by standard techniques, such as with an enzyme linked immunosorbent assay (ELISA) using immobilized polypeptide. If desired, the antibody molecules can be harvested or isolated from the subject (e.g., from the blood or serum of the subject) and further purified by well-known techniques, such as protein A chromatography to obtain the IgG fraction.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) Immunol. Today 4:72), the EBV-hybridoma technique (Cole et al. (1985), Monoclonal Antibodies and Cancer Therapy, Alan R. Liss, Inc., pp. 77-96) or trioma techniques. The technology for producing hybridomas is well known (see generally Current Protocols in Immunology (1994) Coligan et al. (Eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SURFZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92120791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

Recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT Publication No. WO 87/02671; European Patent Application J.84,187; European Patent Application 171,496; European Patent Application 173,494; PCT Publication No. WO 86/01533; U.S. Patent No. 4,816,567; European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. 1987) Cancer Res. 47:999-1005; Wood et al. (1985) Nature 314:446-449; and Shaw et al. (1988) J. Natl. Cancer Inst. 80:1553-1559); Morrison (1985) Science 229:1202-1207; Oi et al. (1986) Bio/Techniques 4:214; U.S. Patent 5,225,539; Jones et al. (1986) Nature 321:552-525; Verhoeyan et al. (1988) Science 239:1534; and Beidler et al. (1988) J. Imununol, 141:4053-4060.

In embodiments in which an anti-MMPFF peptide antibody is contacted with mesothelial fluid obtained from a patient, the mesothelial fluid can be used in its naturally-expressed state or it can be partially purified or clarified prior to use. For example, mesothelial fluid can be centrifuged using standard methods to substantially remove any sediment therefrom prior to contacting the mesothelial fluid with the antibody. Alternatively, the mesothelial fluid can be filtered or ultrafiltered to reduce its volume or to remove large contaminants. Because MMPFF peptides that occur in mesothelial fluid can be expected to be polypeptides having molecular weights in the range 10,000 - 100,000 Daltons, selection of an appropriate filtration/ultrafiltration is necessary to prevent removal of the MMPFF peptide prior to its detection. By way of example, a relatively large pore filter (e.g., one that permits passage of globular particles having molecular weights of 250,000 to 1 million Daltons can be used to remove particulate material from mesothelial fluid and an ultrafiltration device equipped with a membrane that excludes passage of proteins having molecular weights greater than about 5,000 Daltons can be used to concentrate the filtrate. In this example, occurrence of the MMPFF can be assessed in the retentate in the ultrafiltration device.

Because MMPFF peptides are often relatively abundant in mesothelial fluid of patients and because many of the methods (e.g., the immunological methods) described herein are relatively sensitive, concentration of mesothelial fluid is usually not necessary to detect occurrence of MMPFF peptides in mesothelial fluid of patients. In situations in which clarification of mesothelial fluid is desired, centrifugation can be preferable, since the potential that MMPFF peptides present in the mesothelial fluid will become bound with or enmeshed within the filter medium is not present.

One preferred method for assessing occurrence of an MMPFF peptide in mesothelial fluid of a patient is the procedure commonly known as a "sandwich ELISA" assay. (ELISA is an abbreviation for enzyme-linked immunosorbent assay.) In this , I method, an antibody is bound to a substrate, such as a glass bead or the bottom surface of a plastic multi-well assay plate. This antibody is designated a 'capture' antibody. Raw, clarified, or purified mesothelial fluid from the patient is contacted with the substrate under conditions (e.g., low concentrations of salt and detergents and non-protein-denaturing conditions), so that any MMPFF peptide present in the mesothelial fluid binds specifically with the capture antibody. The substrate is optionally rinsed with a fluid that does not comprise the MMPFF peptide to remove residual mesothelial fluid and any non-bound MMPFF. The substrate is then contacted with a second antibody that specifically binds with the MMPFF peptide at an epitope independent of the epitope at which the capture antibody binds. The second antibody is either detectably labeled or linked with either a ligand or a receptor of the ligand. Either way, binding of the second antibody is detected (and, optionally, quantitated) after rinsing the substrate with a fluid that does not comprise the second antibody. Detection of the second antibody is indicative of the presence in the mesothelial fluid of the MMPFF peptide. If the amount of the second antibody is quantitated, then the amount of the MMPFF peptide in the mesothelial fluid can be quantitated, for example, by comparison of the amount of second antibody detected with measurements made using control samples containing known amounts of the MMPFF.

Detection an antibody can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin / biotin and avidin / biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Use of standardized assay apparatus permits automation of the method described herein. For example, many different standardized assay containers are known, such as 24-, 48-, 96-, and 384-well plastic plates. Where these containers are adapted to fit a robotic apparatus, automated analysis of samples can be achieved, permitting high-throughput screening of many samples in a relatively short time. Automated assay apparatus and containers adapted for their use in computer-controlled assays are known in the art and readily adapted for the kits and methods described herein.

Another type of immunological assay that can be used to assess occurrence of an MMPFF peptide in mesothelial fluid obtained from a patient is commonly designated a 'competition' assay. In this assay, an antibody that binds specifically with the MMPFF peptide is fixed to a substrate. Raw, clarified, or purified mesothelial fluid is contacted with the substrate (preferably for a period of minutes or hours), so that any MMPFF peptide present in the mesothelial fluid binds with the antibody. A labeled ligand (e.g., a labeled version of the same MMPFF peptide or another MMPFF peptide) that binds specifically with the antibody is then contacted with the substrate (preferably for a period of minutes or hours). The amount of labeled ligand bound with the substrate is assessed after rinsing the substrate with a fluid that does not comprise the label or the labeled ligand. The amount of label bound with the substrate is compared with the amount of label bound with an otherwise identically treated substrate that is not contacted with the patient's mesothelial fluid. The difference between the two amounts of bound label represents the amount of MMPFF peptide bound to the substrate, and is indicative of the amount of the MMPFF peptide that was present in the mesothelial fluid sample applied to the substrate.

When an immunological competition assay is used, the labeled ligand of the antibody bound with the substrate should be as nearly identical to the MMPFF peptide known or expected to be present in the patient's mesothelial fluid as possible. For example, the ligand and the MMPFF peptide should have or comprise the same amino acid sequence. Similarly, if the MMPFF peptide that may occur in the patient's mesothelial fluid is expected to by glycosylated, then the ligand should be glycosylated in the same way, at the same position, and to the same extent. In this way, affinity differences of the antibody for the ligand and for the MMPFF peptide can be minimized and the accuracy of the competition assay can be improved.

The kits and methods described herein can be used alone to assess occurrence of an MMPFF peptide in mesothelial fluid of a human patient, and such occurrence is indicative of the biochemical and pathological status of the components of the corresponding mesothelial cavity. However, a greater number of pathological or biochemical states can be detected and greater confidence in the status of the mesothelial cavity contents can be achieved if occurrence of more than one marker of the status of the mesothelial cavity content is assessed in the patient's mesothelial fluid. Any additional marker(s) can be a marker normally found in mesothelial fluid, a marker normally found in blood, or a marker normally found in both mesothelial fluid and blood. Thus, the kits and methods described herein for assessing occurrence of MMPFF peptides in mesothelial fluid can be used in conjunction with kits and methods of detecting the same or other MMPFF peptides in serum.

The kits and methods described herein for assessing occurrence of a MMPFF peptide in mesothelial fluid obtained from a patient can be used to assess the likelihood that the patient will develop a pathological condition for a component of the corresponding mesothelial cavity. Detection of occurrence of an MMPFF peptide in a patient's mesothelial fluid is an indication that the patient is more likely to develop such a pathological fluid than an otherwise identical patient in whose mesothelial fluid the MMPFF does not occur. It is recognized that the difference between being afflicted with a condition, the earliest stages of the condition, and enhanced susceptibility to the condition may be substantially indistinguishable. Nonetheless, the outcome of each of these processes is development of the condition to the detriment of the patient's health. For this reason, assessment of any of these states using the kits and methods disclosed herein is useful.

Anti-MMPFF peptide antibodies can be used diagnostically or prognostically to monitor MMPFF levels in mesothelial fluid as part of a clinical testing procedure, e.g., to, for example, determine the efficacy of a given treatment regimen.

The kits and methods described herein can be used to determine whether an agent (e.g., an agonist, antagonist, peptidomimetic, protein, peptide, nucleic acid, small molecule, or other drug candidate) can be administered to a subject in order to alleviate, inhibit, reverse, or prevent pathological conditions of a component of the corresponding mesothelial cavity component. For example, such methods can be used to determine whether a patient can be effectively treated using a specific agent or class of agents.

Kits for Assessing Occurrence of MMPFF Peptides in a Mesothelial Fluid

The invention also encompasses kits for detecting occurrence of an MMPFF in a mesothelial fluid sample obtained from a mammal (e.g., a human patient). The kit comprises a first agent for binding the MMPFF peptide, a second agent for assessing binding of the MMPFF peptide with the agent, and an instructional material that describes contacting mesothelial fluid with the first agent. These kits can be used to determine if a subject is suffering from or is at increased risk of developing a pathological condition of a mesothelial cavity component. For example, the kit can comprise a labeled compound or agent capable of detecting the MMPFF peptide in a mesothelial fluid sample. The kit can also, or alternatively, contain means for determining the amount of the MMPFF peptide in the sample. Kits can include instructions for assessing whether the tested subject is suffering from or is at risk of developing a pathological condition if the amount of the MMPFF peptide is above or below a normal level (e.g., an absorbance measurement of at least 0.2 at 450 nanometers using the methodology set forth in Example 1).

For antibody-based kits, the kit can comprise, for example: (1) a first antibody (e.g., attached to a solid support) which specifically binds with an MMPFF peptide and, optionally, (2) a second, different antibody which specifically binds with either the MMPFF peptide (e.g., at a epitope distinct from the epitope at which the first antibody binds) or the first antibody and is conjugated with a detectable agent. Alternatively, the kit can comprise the first antibody and a labeled ligand of the first antibody. After contacting the first antibody with a patient's mesothelial fluid sample, binding of the labeled ligand with the first antibody can be assessed in a competition-type assay, as described herein.

The kits can further comprise reagents and/or instructions for assessing occurrence in mesothelial fluid or serum of the patient another marker indicative of occurrence of a pathological condition in the patient.

Examples

The invention is now described with reference to the following Examples. These Examples are provided for the purpose of illustration only, and the invention is not limited to these Examples, but rather encompasses all variations which are evident as a result of the teaching provided herein.

Example 1

Sandwich ELISA Assay for Assessing MMPFF in a Sample

A typical assay used to detect an MMPFF in a mesothelial fluid sample is now described. Assays were performed in a 96-well clear, flat-bottom microtiter plate (Nunc) that had been previously coated with 100 microliters of capture antibody (MAb, 4H3 as described in Scholler et al., 1999, Proc, Natl. Acad. Sci. USA 96(12):11531-11536 and in PCT publication WO 00/50900) at a concentration of 5 micrograms per milliliter in carbonate-bicarbonate buffer, pH 9.6 overnight at 4°C. Each well was then blocked by filling it with a 3% (w/v) suspension of bovine serum albumin (BSA) for 2 hours at room temperature, after which time the BSA suspension was removed from the wells.

Standard curves were prepared using a fusion protein comprising a portion of the mesothelin amino acid sequence (i.e., residues 294-628) fused to an immunoglobulin (Ig) constant region using a described vector encoding a human Ig sequence (as described in PCT publication number WO 00/50900 and in Hollenbaugh et al., 1995, J. Immunol. Meth. 188:1-7). This fusion protein was designated hIgG. Antigen was diluted to 10, 5, 2.5, 1.0, and 0.5 nanograms per milliliter. Sample diluent used for standard dilutions as well as detection antibodies was a 7% (w/v) suspension of BSA.

To perform the assay, 100 microliters of each standard dilution, controls, a mesothelial fluid sample, or a diluted mesothelial fluid sample was added to individual wells. The plate was incubated for 1 hour at room temperature, and the wells were thereafter washed with tris-buffered saline (pH 7.8 to 8) containing 0.05% (v/v) TWEEN® 20 surfactant. 100 Microliters of a 200 nanogram per milliliter suspension of biotinylated monoclonal antibody OV569 (as described in Scholler et al., 1999, Proc, Natl. Acad. Sci, USA 96(20):11531-11536 and in PCT publication number WO 00150900) was added to each well at a concentration of 1 microgram per milliliter. After incubating the plate for 1 - hour at room temperature and thereafter washing each well with tris-buffered saline (pH 7.8 to 8) containing 0.05% (v/v) TWEEN® 20 surfactant, 50 microliters of a 1:5000 dilution of Streptavidin-horseradish peroxidase (obtained from Southern Biotechnology Associates, Inc., Birmingham, AL, catalog no. 7100-05) at was added to each well, and the plate was incubated for 1 hour at room temperature. 100 Microliters of 3,3',5,5'-tetramethylbenzidine (TMB; a chromogenic substrate for horseradish peroxidase; obtained from Kirkegaard & Perry Laboratories, Inc., Gaithersburg, MD) was added to each well to generate signal. The plate was incubated for 15 minutes at room temperature before adding 100 microliters of TMB Stop solution (0.1 normal sulfuric acid; obtained from Kirkegaard & Perry Laboratories, Inc., Gaithersburg, MD) to halt the peroxidase reaction. Blue color generated by the action of the peroxidase on TMB was assessed using a Molecular Devices SpectraMax® Plus 384 spectrophotometric plate reader (model number 02523) to measure the absorbance at 450 nanometers.

Example 2

Detection of an MMPFF Peptide in Peritoneal Dialysis Fluid Samples Obtained from Patients

Peritoneal dialysis is one of the treatments for end-stage renal disease and serves as a replacement for lost kidney function. Potential applications for the invention include, but are not restricted to, the ability to monitor the status of the peritoned cavity and determination of membrane integrity.

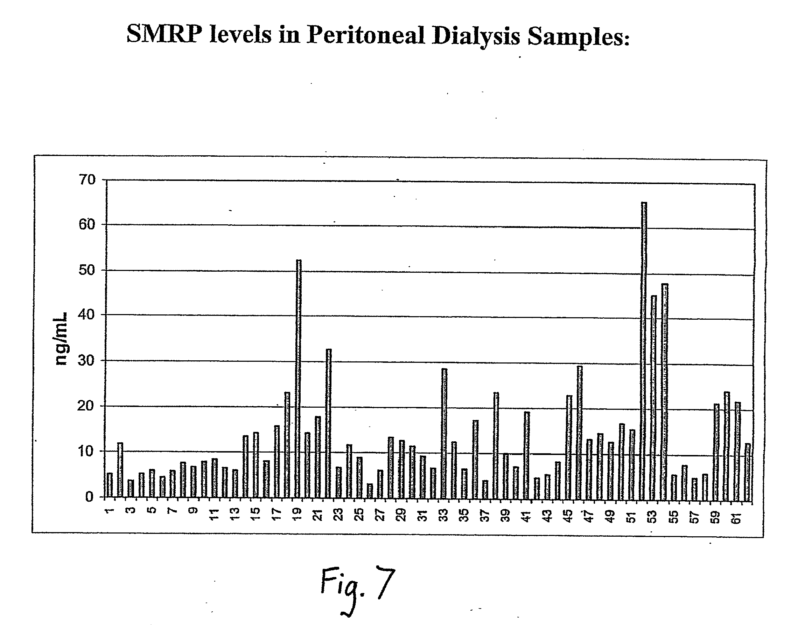

62 dialysis samples were obtained from 34 patients on different dates (ranging from 1, to 6 draws per patient). Samples were diluted 1:10 in dialysis solution. Dialysis solution was run as a negative control. 100 Microliters of each sample dilution was tested in the assay described in Example 1. The results, displayed in Figure 7, indicate that MMPFF peptide is useful as a marker in paritoneal dialysis fluid.

The application inter alia comprises the following aspects:
A1. A method of monitoring a biochemical or pathological status of a mesothelial cavity component in a patient, the method comprising assessing occurrence of a mesothelin/megakaryocyte potentiating factor family (MMPFF) peptide in a mesothelial fluid obtained from the patient, whereby occurrence of the MMPFF peptide in the mesothelial fluid is an indication of the biochemical or pathological status of a components of the corresponding mesothelial cavity.
A2. The method of aspect A1, wherein the mesothelial cavity is a pleural cavity.
A3. The method of aspect A1, wherein the mesothelial cavity is the peritoneum.
A4. The method of aspect A1, wherein the mesothelial cavity is the pericardium.
A5. The method of aspect A1, wherein occurrence of the MMPFF peptide in the mesothelial fluid is assessed by contacting the mesothelial fluid with a first antibody that binds specifically with the MMPFF peptide and assessing whether the MMPFF peptide has bound with the first antibody.
A6. The method of aspect A5, wherein the mesothelial fluid is centrifuged to substantially remove any sediment therefrom prior to contacting the mesothelial fluid with the first antibody.
A7. The method of aspect A5, wherein the first antibody is bound to a substrate.
A8. The method of aspect A7, wherein the substrate is a plastic container.
A9. The method of aspect A8, wherein the container is a multi-well plate.
A10. The method of aspect A9, wherein the plate is adapted for automated analysis by a robotic apparatus.
A11. The method of aspect A5, wherein binding of the MMPFF peptide and the first antibody is assessed by contacting the first antibody with a second antibody that binds specifically with the MMPFF peptide after contacting the first antibody with the mesothelial fluid and assessing co-localization of the first and second antibodies.
A12. The method of aspect A11, wherein the mesothelial fluid is contacted with the first antibody for at least 10 minutes before contacting the first antibody with the second antibody.
A13. The method of aspect A11, wherein the mesothelial fluid is contacted with the first antibody for at least 60 minutes before contacting the first antibody with the second antibody.
A14. The method of aspect A11, wherein the second antibody is detectably labeled.
A15. The method of aspect A14, wherein the second antibody is linked with a compound selected from the group consisting of an enzyme, a radionuclide, a fluorophore, and a chromophore.
A16. The method of aspect A11, wherein the second antibody is linked to a ligand and wherein co-localization of the first and second antibodies is assessed by contacting the second antibody with a compound comprising (i) a receptor that binds with the ligand and (ii) a detectable label.
A17. The method of aspect A16, wherein the label is selected from the group consisting of an enzyme, a radionuclide, a fluorophore, and a chromophore.
A18. The method of aspect A16, wherein the ligand is biotin and the receptor is an avidin.
A19. The method of aspect A18, wherein the avidin is streptavidin.
A20. The method of aspect A19, wherein the label is an enzyme.
A21. The method of aspect A20, wherein the enzyme is horseradish peroxidase.
A22. The method of aspect A21, wherein co-localization of the first and second antibodies is assessed in the presence of 3,3',5,5'-tetramethylbenzidine.
A23. The method of aspect A5, wherein the first antibody is contacted with a labeled substrate of the first antibody after contacting the first antibody with the mesothelial fluid and comparing the amount of labeled ligand that binds with the first antibody that has been contacted with the mesothelial fluid and the amount of labeled ligand that binds with an equivalent amount of the first antibody that has not been contacted with the mesothelial fluid.
A24. The method of aspect A23, wherein the labeled ligand has the same amino acid sequence as the MMPFF peptide.
A25. The method of aspect A24, wherein the glycosylation state of the labeled ligand is the same as the glycosylation state of the MMPFF peptide.
A26. The method of aspect A1, wherein the MMPFF peptide is glycosylated.
A27. The method of aspect A1, wherein the amino acid sequence of the MMPFF peptide comprises at least 10 consecutive residues of a sequence selected from the group consisting of SEQ ID NOs: 1-5.
A28. The method of aspect A27, wherein the amino acid sequence of the MMPFF peptide comprises at least 20 consecutive residues of a sequence selected from the group consisting of SEQ ID NOs: 1-5.
A29. The method of aspect A27, wherein the amino acid sequence of the MMPFF peptide comprises at least 50 consecutive residues of a sequence selected from the group consisting of SEQ ID NOs: 1-5.
A30. The method of aspect A27, wherein the amino acid sequence of the MMPFF peptide comprises at least 200 consecutive residues of a sequence selected from the group consisting of SEQ ID NOs: 1-5.
A31. The method of aspect A27, wherein the amino acid sequence of the MMPFF peptide comprises a sequence selected from the group consisting of SEQ ID NOs: 1-5.
A32. The method of aspect A27, wherein the amino acid sequence of the MMPFF peptide comprises at least 10 consecutive residues of SEQ ID NO: 4.
A33. The method of aspect A27, wherein the amino acid sequence of the MMPFF peptide comprises at least 10 consecutive residues of SEQ ID NO: 5.
A34. The method of aspect A1, wherein the MMPFF peptide comprises a portion of at least 20 consecutive amino acid residues and wherein the amino acid sequence of the portion is at least 90% identical to 20 consecutive residues of a sequence selected from the group consisting of SEQ ID NOs: 1-5.
A35. The method of aspect A34, wherein the MMPFF peptide comprises a portion of at least 20 consecutive amino acid residues and wherein the amino acid sequence of the portion is at least 90°l° identical to 20 consecutive residues of a sequence selected from the group consisting of SEQ ID NOs: 4 and 5.
A36. The method of aspect A1, further comprising assessing occurrence of a second marker of a pathological status of a mesothelial cavity component in the mesothelial fluid of the patient, whereby occurrence of the second marker in the mesothelial fluid is also an indication that the patient is afflicted with a disorder of a component of the corresponding mesothelial cavity.
A37. The method of aspect A1, further comprising assessing occurrence of a second marker in the serum of the patient, whereby occurrence of the second marker in the serum is also an indication that the patient is afflicted with the disorder.
A38. The method of aspect A1, further comprising assessing occurrence of the MMPFF peptide in the serum of the patient, whereby occurrence of the MMPFF peptide in the serum is also an indication that the patient is afflicted with the disorder.
B. A method of assessing the likelihood that a patient will develop a pathological status of a mesothelial cavity component, the method comprising assessing occurrence of a mesothelin/megakaryocyte potentiating factor family (MMPFF) peptide in mesothelial fluid obtained from the patient, whereby occurrence of the MMPFF peptide in the mesothelial fluid is an indication that the patient is more likely to develop the pathological status than an otherwise identical patient in whose corresponding mesothelial fluid the MMPFF does not occur.
C. A method of assessing the efficacy of a therapy for a pathological status of a mesothelial cavity component in a patient, the method comprising assessing the amount of a mesothelin/megakaryocyte potentiating factor family (MMPFF) peptide in mesothelial fluid obtained from the patient following the therapy and comparing that amount with the amount of the MMPFF in mesothelial fluid obtained from the patient before the therapy, whereby a lower amount of the MMPFF peptide in the mesothelial fluid following the therapy is an indication that the therapy is efficacious.
D41. A kit for assessing occurrence of a mesothelin/megakaryocyte potentiating factor family (MMPFF) peptide in mesothelial fluid obtained from a patient, the kit comprising a first agent for binding the MMPFF peptide and an instructional material that describes contacting mesothelial fluid with the first agent.
D42. The kit of aspect D41, wherein the first agent is an antibody.
D43. The kit of aspect D42, wherein the antibody is bound to a thin metal film.
D44. The kit of aspect D43, wherein the film is adapted for analysis in a surface plasmon resonance apparatus.
D45. The kit of aspect D41, further comprising a second agent for assessing binding of the MMPFF peptide with the first agent.
D46. The kit of aspect D45, wherein the second agent is a detectably labeled ligand of the first agent.
D47. The kit of aspect D46, wherein the first agent is an antibody and the ligand is an MMPFF peptide.
D48. The kit of aspect D46, wherein the ligand is labeled with a compound selected from the group consisting of an enzyme, a radionuclide, a fluorophore, and a chromophore.
D49. The kit of aspect D45, wherein the second agent is a detectably labeled antibody.
D50. The kit of aspect D49, wherein the antibody is a biotinylated antibody.
D51. The kit of aspect D49, wherein the antibody is labeled with a compound selected from the group consisting of an enzyme, a radionuclide, a fluorophore, and a chromophore.
D52. The kit of aspect D45, wherein the first agent is an antibody that binds with an epitope of the MMPFF that is distinct from the epitope that binds with the detectably labeled antibody.
D53. The kit of aspect D41, further comprising a device for centrifuging a mesothelial fluid sample obtained from the patient.
D54. The kit of aspect D41, wherein the first agent is bound to a substrate.
D55. The kit of aspect D54, wherein the substrate is a plastic container.
D56. The kit of aspect D55, wherein the container is a multi-well plate.
D57. The kit of aspect D56, wherein the plate is adapted for automated analysis by a robotic apparatus.
D58. The kit of aspect D55, wherein the first agent is an antibody.
D59. The kit of aspect D58, wherein the second agent is second antibody.
D60. The kit of aspect D59, wherein the second antibody is linked to a ligand.
D61. The kit of aspect D60, further comprising a compound comprising (i) a receptor that binds with the ligand and (ii) a detectable label.
D62. The kit of aspect D61, wherein the label is selected from the group consisting of an enzyme, a radionuclide, a fluorophore, and a chromophore.
D63. The kit of aspect D62, wherein the ligand is biotin and the receptor is an avidin.
D64. The kit of aspect D63, wherein the avidin is streptavidin.
D65. The kit of aspect D64, wherein the label is an enzyme.
D66. The kit of aspect D65, wherein the enzyme is horseradish peroxidase.
D67. The kit of aspect D66, further comprising 3,3',5,5'-tetramethylbenzidine.
D68. The kit of aspect D41, further comprising a control MMPFF peptide, wherein the amino acid sequence of the control peptide comprises at least 10 consecutive residues of a sequence selected from the group consisting of SEQ ID NOs: 1-5.
D69. The kit of aspect D68, wherein the amino acid sequence of the control peptide comprises at least 20 consecutive residues of a sequence selected from the group consisting of SEQ ID NOs: 1-5.
D70. The kit of aspect D68, wherein the amino acid sequence of the control peptide comprises at least 50 consecutive residues of a sequence selected from the group consisting of SEQ ID NOs: 1-5.
D71. The kit of aspect D68, wherein the amino acid sequence of the control peptide comprises at least 200 consecutive residues of a sequence selected from the group consisting of SEQ ID NOs: 1-5.
D72. The kit of aspect D68, wherein the amino acid sequence of the control peptide comprises a sequence selected from the group consisting of SEQ ID NOs: 1-5.
D73. The kit of aspect D68, wherein the amino acid sequence of the control peptide comprises at least 10 consecutive residues of SEQ ID NO: 4.
D74. The kit of aspect D68, wherein the amino acid sequence of the control peptide comprises at least 10 consecutive residues of SEQ ID NO: 5.
D75. The kit of aspect D41, further comprising a control MMPFF peptide comprises a portion of at least 20 consecutive amino acid residues and wherein the amino acid sequence of the portion is at least 90% identical to 20 consecutive residues of a sequence selected from the group consisting of SEQ ID NOs: 1-5.
D76. The kit of aspect D75, wherein the control peptide comprises a portion of at least 20 consecutive amino acid residues and wherein the amino acid sequence of the portion is at least 90% identical to 20 consecutive residues of a sequence selected from the group consisting of SEQ ID NOs: 4 and 5.
D77. The kit of aspect D41, further comprising a reagent for assessing occurrence of a second marker of a pathological status of a component of the corresponding mesothelial cavity in the mesothelial fluid of the patient.
D78. The kit of aspect D41, further comprising a reagent for assessing occurrence of a second of a pathological status of a component of the corresponding mesothelial cavity in the serum of the patient.

## Claims

1. A method of monitoring a biochemical or pathological status of a mesothelial cavity component in a patient, the method comprising assessing occurrence of a mesothelin/megakaryocyte potentiating factor family (MMPFF) peptide in a mesothelial fluid obtained from the patient, whereby occurrence of the MMPFF peptide in the mesothelial fluid is an indication of the biochemical or pathological status of a components of the corresponding mesothelial cavity.

2. The method of claim 1, wherein occurrence of the MMPFF peptide in the mesothelial fluid is assessed by contacting the mesothelial fluid with a first antibody that binds specifically with the MMPFF peptide and assessing whether the MMPFF peptide has bound with the first antibody.

3. The method of claim 2, wherein the first antibody is bound to a substrate, the substrate being a plastic container, preferably a multi-well plate.

4. The method of claim 2, wherein binding of the MMPFF peptide and the first antibody is assessed by contacting the first antibody with a second antibody that binds specifically with the MMPFF peptide after contacting the first antibody with the mesothelial fluid and assessing co-localization of the first and second antibodies.

5. The method of claim 4, wherein the second antibody is detectably labeled and is preferably linked with a compound selected from the group consisting of an enzyme, a radionuclide, a fluorophore, and a chromophore; or wherein the second antibody is linked to a ligand and wherein co-localization of the first and second antibodies is assessed by contacting the second antibody with a compound comprising (i) a receptor that binds with the ligand and (ii) a detectable label, the label preferably being selected from the group consisting of an enzyme, a radionuclide, a fluorophore, and a chromophore, the ligand preferably being biotin and the receptor preferably being an avidin.

6. The method of claim 2, wherein the first antibody is contacted with a labeled substrate of the first antibody after contacting the first antibody with the mesothelial fluid and comparing the amount of labeled ligand that binds with the first antibody that has been contacted with the mesothelial fluid and the amount of labeled ligand that binds with an equivalent amount of the first antibody that has not been contacted with the mesothelial fluid, wherein the labeled ligand has the same amino acid sequence as the MMPFF peptide, and wherein the glycosylation state of the labeled ligand preferably is the same as the glycosylation state of the MMPFF peptide.

7. The method of any of claims 1 to 6, wherein the amino acid sequence of the MMPFF peptide comprises at least 10 consecutive residues of either SEQ ID NO: 4 or SEO ID NO: 5.

8. The method of any of claims 1 to 6, wherein the MMPFF peptide comprises a portion of at least 20 consecutive amino acid residues and wherein the amino acid sequence of the portion is at least 90% identical to 20 consecutive residues of a sequence selected from the group consisting of SEQ ID NOs: 1-5, and preferably selected from the group consisting of SEQ ID NOs: 4 and 5.

9. The method of any of the preceding claims, further comprising assessing occurrence of a second marker of a pathological status of a mesothelial cavity component in the mesothelial fluid of the patient, whereby occurrence of the second marker in the mesothelial fluid is also an indication that the patient is afflicted with a disorder of a component of the corresponding mesothelial cavity.

10. A method of assessing the likelihood that a patient will develop a pathological status of a mesothelial cavity component, the method comprising assessing occurrence of a mesothelin/megakaryocyte potentiating factor family (MMPFF) peptide in mesothelial fluid obtained from the patient, whereby occurrence of the MMPFF peptide in the mesothelial fluid is an indication that the patient is more likely to develop the pathological status than an otherwise identical patient in whose corresponding mesothelial fluid the MMPFF does not occur.

11. A method of assessing the efficacy of a therapy for a pathological status of a mesothelial cavity component in a patient, the method comprising assessing the amount of a mesothelin/megakaryocyte potentiating factor family (MMPFF) peptide in mesothelial fluid obtained from the patient following the therapy and comparing that amount with the amount of the MMPFF in mesothelial fluid obtained from the patient before the therapy, whereby a lower amount of the MMPFF peptide in the mesothelial fluid following the therapy is an indication that the therapy is efficacious.

12. A kit for assessing occurrence of a mesothelin/megakaryocyte potentiating factor family (MMPFF) peptide in mesothelial fluid obtained from a patient, the kit comprising a first agent for binding the MMPFF peptide and an instructional material that describes contacting mesothelial fluid with the first agent, wherein the first agent is an antibody, wherein the antibody is preferably bound to a thin metal film, and the film can be adapted for analysis in a surface plasmon resonance apparatus.

13. The kit of claim 12, further comprising a second agent for assessing binding of the MMPFF peptide with the first agent.

14. The kit of claim 27, wherein the second agent is (a) a detectably labeled ligand of the first agent, or is (b) a detectably labeled antibody, wherein in variant (a), the first agent is an antibody and the ligand is an MMPFF peptide, or the ligand is labeled with a compound selected from the group consisting of an enzyme, a radionuclide, a fluorophore, and a chromophore, or wherein in variant (b), the antibody is a biotinylated antibody, or the antibody is labeled with a compound selected from the group consisting of an enzyme, a radionuclide, a fluorophore, and a chromophore.

15. The kit of claim 13, wherein the first agent is an antibody that binds with an epitope of the MMPFF that is distinct from the epitope that binds with the detectably labeled antibody.

16. The kit of any of claims 12 to 15, further comprising a control MMPFF peptide, wherein the amino acid sequence of the control peptide comprises a sequence selected from the group consisting of SEQ ID NOs: 1-5, the sequence preferably having at least 10 consecutive residues of SEQ ID NO: 4 or at least 10 consecutive residues of SEQ ID NO: 5.

17. The kit of any of claims 12 to 16, further comprising a reagent for assessing occurrence of a second marker of a pathological status of a component of the corresponding mesothelial cavity in the mesothelial fluid or in the serum of the patient.


SEQUENCE LISTING

<110> O'SHANNESSY, Daniel J.
      SARDESAI, Niranjan
      SOMERS, Elizabeth B.

<120> Detection of Urinary Mesothelin-/Megakaryocyte Potentiating
      Factor-Related Peptides in Peritoneal Fluid for Assessment of the
       Peritoneum and the Peritoneal Cavity

<130> D4496-00096

<140> EP 05 71 2014.9
<141> 2005-01-21

<150> US 11/040,240
<151> 2005-01-21

<150> US 60/538,072
<151> 2004-01-21

<160> 5

<170> PatentIn version 3.3

<210> 1
<211> 628
<212> PRT
<213> Homo sapiens

<400> 1

Met Ala Leu Gln Arg Leu Asp Pro Cys Trp Ser Cys Gly Asp Arg Pro
1               5                   10                  15

Gly Ser Leu Leu Phe Leu Leu Phe Ser Leu Gly Trp Val His Pro Ala
            20                  25                  30

Arg Thr Leu Ala Gly Glu Thr Gly Thr Glu Ser Ala Pro Leu Gly Gly
        35                  40                  45

Val Leu Thr Thr Pro His Asn Ile Ser Ser Leu Ser Pro Arg Gln Leu
    50                  55                  60

Leu Gly Phe Pro Cys Ala Glu Val Ser Gly Leu Ser Thr Glu Arg Val
65                  70                  75                  80

Arg Glu Leu Ala Val Ala Leu Ala Gln Lys Asn Val Lys Leu Ser Thr
                85                  90                  95

Glu Gln Leu Arg Cys Leu Ala His Arg Leu Ser Glu Pro Pro Glu Asp
            100                 105                 110

Leu Asp Ala Leu Pro Leu Asp Leu Leu Leu Phe Leu Asn Pro Asp Ala
        115                 120                 125

Phe Ser Gly Pro Gln Ala Cys Thr Arg Phe Phe Ser Arg Ile Thr Lys
    130                 135                 140

Ala Asn Val Asp Leu Leu Pro Arg Gly Ala Pro Glu Arg Gln Arg Leu
145                 150                 155                 160

Leu Pro Ala Ala Leu Ala Cys Trp Gly Val Arg Gly Ser Leu Leu Ser
                165                 170                 175

Glu Ala Asp Val Arg Ala Leu Gly Gly Leu Ala Cys Asp Leu Pro Gly
            180                 185                 190

Arg Phe Val Ala Glu Ser Ala Glu Val Leu Leu Pro Arg Leu Val Ser
        195                 200                 205

Cys Pro Gly Pro Leu Asp Gln Asp Gln Gln Glu Ala Ala Arg Ala Ala
    210                 215                 220

Leu Gln Gly Gly Gly Pro Pro Tyr Gly Pro Pro Ser Thr Trp Ser Val
225                 230                 235                 240

Ser Thr Met Asp Ala Leu Arg Gly Leu Leu Pro Val Leu Gly Gln Pro
                245                 250                 255

Ile Ile Arg Ser Ile Pro Gln Gly Ile Val Ala Ala Trp Arg Gln Arg
            260                 265                 270

Ser Ser Arg Asp Pro Ser Trp Arg Gln Pro Glu Arg Thr Ile Leu Arg
        275                 280                 285

Pro Arg Phe Arg Arg Glu Val Glu Lys Thr Ala Cys Pro Ser Gly Lys
    290                 295                 300

Lys Ala Arg Glu Ile Asp Glu Ser Leu Ile Phe Tyr Lys Lys Trp Glu
305                 310                 315                 320

Leu Glu Ala Cys Val Asp Ala Ala Leu Leu Ala Thr Gln Met Asp Arg
                325                 330                 335

Val Asn Ala Ile Pro Phe Thr Tyr Glu Gln Leu Asp Val Leu Lys His
            340                 345                 350

Lys Leu Asp Glu Leu Tyr Pro Gln Gly Tyr Pro Glu Ser Val Ile Gln
        355                 360                 365

His Leu Gly Tyr Leu Phe Leu Lys Met Ser Pro Glu Asp Ile Arg Lys
    370                 375                 380

Trp Asn Val Thr Ser Leu Glu Thr Leu Lys Ala Leu Leu Glu Val Asp
385                 390                 395                 400

Lys Gly His Glu Met Ser Pro Gln Ala Pro Arg Arg Pro Leu Pro Gln
                405                 410                 415

Val Ala Thr Leu Ile Asp Arg Phe Val Lys Gly Arg Gly Gln Leu Asp
            420                 425                 430

Lys Asp Thr Leu Asp Thr Leu Thr Ala Phe Tyr Pro Gly Tyr Leu Cys
        435                 440                 445

Ser Leu Ser Pro Glu Glu Leu Ser Ser Val Pro Pro Ser Ser Ile Trp
    450                 455                 460

Ala Val Arg Pro Gln Asp Leu Asp Thr Cys Asp Pro Arg Gln Leu Asp
465                 470                 475                 480

Val Leu Tyr Pro Lys Ala Arg Leu Ala Phe Gln Asn Met Asn Gly Ser
                485                 490                 495

Glu Tyr Phe Val Lys Ile Gln Ser Phe Leu Gly Gly Ala Pro Thr Glu
            500                 505                 510

Asp Leu Lys Ala Leu Ser Gln Gln Asn Val Ser Met Asp Leu Ala Thr
        515                 520                 525

Phe Met Lys Leu Arg Thr Asp Ala Val Leu Pro Leu Thr Val Ala Glu
    530                 535                 540

Val Gln Lys Leu Leu Gly Pro His Val Glu Gly Leu Lys Ala Glu Glu
545                 550                 555                 560

Arg His Arg Pro Val Arg Asp Trp Ile Leu Arg Gln Arg Gln Asp Asp
                565                 570                 575

Leu Asp Thr Leu Gly Leu Gly Leu Gln Gly Gly Ile Pro Asn Gly Tyr
            580                 585                 590

Leu Val Leu Asp Leu Ser Val Gln Glu Thr Leu Ser Gly Thr Pro Cys
        595                 600                 605

Leu Leu Gly Pro Gly Pro Val Leu Thr Val Leu Ala Leu Leu Leu Ala
    610                 615                 620

Ser Thr Leu Ala
625

<210> 2
<211> 622
<212> PRT
<213> Homo sapiens

<400> 2

Met Ala Leu Pro Thr Ala Arg Pro Leu Leu Gly Ser Cys Gly Thr Pro
1               5                   10                  15

Ala Leu Gly Ser Leu Leu Phe Leu Leu Phe Ser Leu Gly Trp Val Gln

            20                  25                  30

Pro Ser Arg Thr Leu Ala Gly Glu Thr Gly Gln Glu Ala Ala Pro Leu
        35                  40                  45

Asp Gly Val Leu Ala Asn Pro Pro Asn Ile Ser Ser Leu Ser Pro Arg
    50                  55                  60

Gln Leu Leu Gly Phe Pro Cys Ala Glu Val Ser Gly Leu Ser Thr Glu
65                  70                  75                  80

Arg Val Arg Glu Leu Ala Val Ala Leu Ala Gln Lys Asn Val Lys Leu
                85                  90                  95

Ser Thr Glu Gln Leu Arg Cys Leu Ala His Arg Leu Ser Glu Pro Pro
            100                 105                 110

Glu Asp Leu Asp Ala Leu Pro Leu Asp Leu Leu Leu Phe Leu Asn Pro
        115                 120                 125

Asp Ala Phe Ser Gly Pro Gln Ala Cys Thr Arg Phe Phe Ser Arg Ile
    130                 135                 140

Thr Lys Ala Asn Val Asp Leu Leu Pro Arg Gly Ala Pro Glu Arg Gln
145                 150                 155                 160

Arg Leu Leu Pro Ala Ala Leu Ala Cys Trp Gly Val Arg Gly Ser Leu
                165                 170                 175

Leu Ser Glu Ala Asp Val Arg Ala Leu Gly Gly Leu Ala Cys Asp Leu
            180                 185                 190

Pro Gly Arg Phe Val Ala Glu Ser Ala Glu Val Leu Leu Pro Arg Leu
        195                 200                 205

Val Ser Cys Pro Gly Pro Leu Asp Gln Asp Gln Gln Glu Ala Ala Arg
    210                 215                 220

Ala Ala Leu Gln Gly Gly Gly Pro Pro Tyr Gly Pro Pro Ser Thr Trp
225                 230                 235                 240

Ser Val Ser Thr Met Asp Ala Leu Arg Gly Leu Leu Pro Val Leu Gly
                245                 250                 255

Gln Pro Ile Ile Arg Ser Ile Pro Gln Gly Ile Val Ala Ala Trp Arg
            260                 265                 270

Gln Arg Ser Ser Arg Asp Pro Ser Trp Arg Gln Pro Glu Arg Thr Ile
        275                 280                 285

Leu Arg Pro Arg Phe Arg Arg Glu Val Glu Lys Thr Ala Cys Pro Ser

    290                 295                 300

Gly Lys Lys Ala Arg Glu Ile Asp Glu Ser Leu Ile Phe Tyr Lys Lys
305                 310                 315                 320

Trp Glu Leu Glu Ala Cys Val Asp Ala Ala Leu Leu Ala Thr Gln Met
                325                 330                 335

Asp Arg Val Asn Ala Ile Pro Phe Thr Tyr Glu Gln Leu Asp Val Leu
            340                 345                 350

Lys His Lys Leu Asp Glu Leu Tyr Pro Gln Gly Tyr Pro Glu Ser Val
        355                 360                 365

Ile Gln His Leu Gly Tyr Leu Phe Leu Lys Met Ser Pro Glu Asp Ile
    370                 375                 380

Arg Lys Trp Asn Val Thr Ser Leu Glu Thr Leu Lys Ala Leu Leu Glu
385                 390                 395                 400

Val Asn Lys Gly His Glu Met Ser Pro Gln Val Ala Thr Leu Ile Asp
                405                 410                 415

Arg Phe Val Lys Gly Arg Gly Gln Leu Asp Lys Asp Thr Leu Asp Thr
            420                 425                 430

Leu Thr Ala Phe Tyr Pro Gly Tyr Leu Cys Ser Leu Ser Pro Glu Glu
        435                 440                 445

Leu Ser Ser Val Pro Pro Ser Ser Ile Trp Ala Val Arg Pro Gln Asp
    450                 455                 460

Leu Asp Thr Cys Asp Pro Arg Gln Leu Asp Val Leu Tyr Pro Lys Ala
465                 470                 475                 480

Arg Leu Ala Phe Gln Asn Met Asn Gly Ser Glu Tyr Phe Val Lys Ile
                485                 490                 495

Gln Ser Phe Leu Gly Gly Ala Pro Thr Glu Asp Leu Lys Ala Leu Ser
            500                 505                 510

Gln Gln Asn Val Ser Met Asp Leu Ala Thr Phe Met Lys Leu Arg Thr
        515                 520                 525

Asp Ala Val Leu Pro Leu Thr Val Ala Glu Val Gln Lys Leu Leu Gly
    530                 535                 540

Pro His Val Glu Gly Leu Lys Ala Glu Glu Arg His Arg Pro Val Arg
545                 550                 555                 560

Asp Trp Ile Leu Arg Gln Arg Gln Asp Asp Leu Asp Thr Leu Gly Leu

                565                 570                 575

Gly Leu Gln Gly Gly Ile Pro Asn Gly Tyr Leu Val Leu Asp Leu Ser
            580                 585                 590

Val Gln Glu Ala Leu Ser Gly Thr Pro Cys Leu Leu Gly Pro Gly Pro
        595                 600                 605

Val Leu Thr Val Leu Ala Leu Leu Leu Ala Ser Thr Leu Ala
    610                 615                 620

<210> 3
<211> 398
<212> PRT
<213> Homo sapiens

<400> 3

Val Glu Lys Thr Ala Cys Pro Ser Gly Lys Lys Ala Arg Glu Ile Asp
1               5                   10                  15

Glu Ser Leu Ile Phe Tyr Lys Lys Trp Glu Leu Glu Ala Cys Val Asp
            20                  25                  30

Ala Ala Leu Leu Ala Thr Gln Met Asp Arg Val Asn Ala Ile Pro Phe
        35                  40                  45

Thr Tyr Glu Gln Leu Asp Val Leu Lys His Lys Leu Asp Glu Leu Tyr
    50                  55                  60

Pro Gln Gly Tyr Pro Glu Ser Val Ile Gln His Leu Gly Tyr Leu Phe
65                  70                  75                  80

Leu Lys Met Ser Pro Glu Asp Ile Arg Lys Trp Asn Val Thr Ser Leu
                85                  90                  95

Glu Thr Leu Lys Ala Leu Leu Glu Val Asn Lys Gly His Glu Met Ser
            100                 105                 110

Pro Gln Val Ala Thr Leu Ile Asp Arg Phe Val Lys Gly Arg Gly Gln
        115                 120                 125

Leu Asp Lys Asp Thr Leu Asp Thr Leu Thr Ala Phe Tyr Pro Gly Tyr
    130                 135                 140

Leu Cys Ser Leu Ser Pro Glu Glu Leu Ser Ser Val Pro Pro Ser Ser
145                 150                 155                 160

Ile Trp Ala Val Arg Pro Gln Asp Leu Asp Thr Cys Asp Pro Arg Gln
                165                 170                 175

Leu Asp Val Leu Tyr Pro Lys Ala Arg Leu Ala Phe Gln Asn Met Asn
            180                 185                 190

Gly Ser Glu Tyr Phe Val Lys Ile Gln Ser Phe Leu Gly Gly Ala Pro
        195                 200                 205

Thr Glu Asp Leu Lys Ala Leu Ser Gln Gln Asn Val Ser Met Asp Leu
    210                 215                 220

Ala Thr Phe Met Lys Leu Arg Thr Asp Ala Val Leu Pro Leu Thr Val
225                 230                 235                 240

Ala Glu Val Gln Lys Leu Leu Gly Pro His Val Glu Gly Leu Lys Ala
                245                 250                 255

Glu Glu Arg His Arg Pro Val Arg Asp Trp Ile Leu Arg Gln Arg Gln
            260                 265                 270

Asp Asp Leu Asp Thr Leu Gly Leu Gly Leu Gln Gly Gly Ile Pro Asn
        275                 280                 285

Gly Tyr Leu Val Leu Asp Leu Ser Val Gln Gly Gly Arg Gly Gly Gln
    290                 295                 300

Ala Arg Ala Gly Gly Arg Ala Gly Gly Val Glu Val Gly Ala Leu Ser
305                 310                 315                 320

His Pro Ser Leu Cys Arg Gly Pro Leu Gly Asp Ala Leu Pro Pro Arg
                325                 330                 335

Thr Trp Thr Cys Ser His Arg Pro Gly Thr Ala Pro Ser Leu His Pro
            340                 345                 350

Gly Leu Arg Ala Pro Leu Pro Cys Trp Pro Gln Pro Cys Trp Gly Ser
        355                 360                 365

Pro Pro Gly Gln Glu Gln Ala Arg Val Ile Pro Val Pro Pro Gln Glu
    370                 375                 380

Asn Ser Arg Ser Val Asn Gly Asn Met Pro Pro Ala Asp Thr
385                 390                 395

<210>  4
<211>  307
<212>  PRT
<213>  Homo sapiens

<220>
<221>  misc_feature
<222>  (107)..(107)
<223>  Xaa can be any naturally occurring amino acid

<400>  4

Glu Val Glu Lys Thr Ala Cys Pro Ser Gly Lys Lys Ala Arg Glu Ile

1                   5                   10                  15

Asp Glu Ser Leu Ile Phe Tyr Lys Lys Trp Glu Leu Glu Ala Cys Val
                20                  25                  30

Asp Ala Ala Leu Leu Ala Thr Gln Met Asp Arg Val Asn Ala Ile Pro
            35                  40                  45

Phe Thr Tyr Glu Gln Leu Asp Val Leu Lys His Lys Leu Asp Glu Leu
    50                  55                  60

Tyr Pro Gln Gly Tyr Pro Glu Ser Val Ile Gln His Leu Gly Tyr Leu
65                  70                  75                  80

Phe Leu Lys Met Ser Pro Glu Asp Ile Arg Lys Trp Asn Val Thr Ser
                85                  90                  95

Leu Glu Thr Leu Lys Ala Leu Leu Glu Val Xaa Lys Gly His Glu Met
                100                 105                 110

Ser Pro Gln Ala Pro Arg Arg Pro Leu Pro Gln Val Ala Thr Leu Ile
            115                 120                 125

Asp Arg Phe Val Lys Gly Arg Gly Gln Leu Asp Lys Asp Thr Leu Asp
    130                 135                 140

Thr Leu Thr Ala Phe Tyr Pro Gly Tyr Leu Cys Ser Leu Ser Pro Glu
145                 150                 155                 160

Glu Leu Ser Ser Val Pro Pro Ser Ser Ile Trp Ala Val Arg Pro Gln
                165                 170                 175

Asp Leu Asp Thr Cys Asp Pro Arg Gln Leu Asp Val Leu Tyr Pro Lys
                180                 185                 190

Ala Arg Leu Ala Phe Gln Asn Met Asn Gly Ser Glu Tyr Phe Val Lys
            195                 200                 205

Ile Gln Ser Phe Leu Gly Gly Ala Pro Thr Glu Asp Leu Lys Ala Leu
    210                 215                 220

Ser Gln Gln Asn Val Ser Met Asp Leu Ala Thr Phe Met Lys Leu Arg
225                 230                 235                 240

Thr Asp Ala Val Leu Pro Leu Thr Val Ala Glu Val Gln Lys Leu Leu
                245                 250                 255

Gly Pro His Val Glu Gly Leu Lys Ala Glu Glu Arg His Arg Pro Val
                260                 265                 270

Arg Asp Trp Ile Leu Arg Gln Arg Gln Asp Asp Leu Asp Thr Leu Gly

        275                 280                 285

Leu Gly Leu Gln Gly Gly Ile Pro Asn Gly Tyr Leu Val Leu Asp Leu
    290                 295                 300

Ser Val Gln
305

<210> 5
<211> 299
<212> PRT
<213> Homo sapiens

<220>
<221> misc_feature
<222> (107)..(107)
<223> Xaa can be any naturally occurring amino acid

<400> 5

Glu Val Glu Lys Thr Ala Cys Pro Ser Gly Lys Lys Ala Arg Glu Ile
1               5                   10                  15

Asp Glu Ser Leu Ile Phe Tyr Lys Lys Trp Glu Leu Glu Ala Cys Val
            20                  25                  30

Asp Ala Ala Leu Leu Ala Thr Gln Met Asp Arg Val Asn Ala Ile Pro
        35                  40                  45

Phe Thr Tyr Glu Gln Leu Asp Val Leu Lys His Lys Leu Asp Glu Leu
    50                  55                  60

Tyr Pro Gln Gly Tyr Pro Glu Ser Val Ile Gln His Leu Gly Tyr Leu
65                  70                  75                  80

Phe Leu Lys Met Ser Pro Glu Asp Ile Arg Lys Trp Asn Val Thr Ser
                85                  90                  95

Leu Glu Thr Leu Lys Ala Leu Leu Glu Val Xaa Lys Gly His Glu Met
            100                 105                 110

Ser Pro Gln Val Ala Thr Leu Ile Asp Arg Phe Val Lys Gly Arg Gly
        115                 120                 125

Gln Leu Asp Lys Asp Thr Leu Asp Thr Leu Thr Ala Phe Tyr Pro Gly
    130                 135                 140

Tyr Leu Cys Ser Leu Ser Pro Glu Glu Leu Ser Ser Val Pro Pro Ser
145                 150                 155                 160

Ser Ile Trp Ala Val Arg Pro Gln Asp Leu Asp Thr Cys Asp Pro Arg
                165                 170                 175

Gln Leu Asp Val Leu Tyr Pro Lys Ala Arg Leu Ala Phe Gln Asn Met
            180                 185                 190

Asn Gly Ser Glu Tyr Phe Val Lys Ile Gln Ser Phe Leu Gly Gly Ala
        195                 200                 205

Pro Thr Glu Asp Leu Lys Ala Leu Ser Gln Gln Asn Val Ser Met Asp
    210                 215                 220

Leu Ala Thr Phe Met Lys Leu Arg Thr Asp Ala Val Leu Pro Leu Thr
225                 230                 235                 240

Val Ala Glu Val Gln Lys Leu Leu Gly Pro His Val Glu Gly Leu Lys
                245                 250                 255

Ala Glu Glu Arg His Arg Pro Val Arg Asp Trp Ile Leu Arg Gln Arg
            260                 265                 270

Gln Asp Asp Leu Asp Thr Leu Gly Leu Gly Leu Gln Gly Gly Ile Pro
        275                 280                 285

Asn Gly Tyr Leu Val Leu Asp Leu Ser Val Gln
    290                 295